# EUROPEAN PATENT APPLICATION

(11) **EP 1 352 957 A1**
(43) Date of publication of application: **15.10.2003**
(21) Application number: 03007881.0
(22) Date of filing: 07.04.2003
(51) Int. Cl.: C12N 11/08, C08L 63/00

(54) **Carriers for covalent immobilization of enzymes**

(30) Priority: 08.04.2002 IT MI20020729
(71) Applicant: Resindion S.R.L., 20082 Binasco (IT)
(72) Inventor: Tam, Andrea, 20082 Binasco (Milano) (IT); Re, Daniele, 20082 Binasco (Milano) (IT); Caimi, Paolo, 20082 Binasco (Milano) (IT); Daminati, Moreno, 20082 Binasco (Milano) (IT)
(74) Representative: Forattini, Amelia

(57) **Abstract**

The present invention relates to a carrier for multipoint covalent immobilization of enzymes on a hydrophilic surface by virtue of an initial ionic adsorption. The carrier according to the invention has an inner layer of protic groups and an outer layer of epoxide groups. The invention also relates to a method for synthesizing the carrier according to the invention, starting from a copolymer or monomer that is subsequently copolymerized. The invention also relates to the use of the carrier according to the invention to immobilize enzymes on a surface by ionic adsorption and covalent bond.

## Description

The present invention relates to a new class of carriers for promoting multipoint covalent immobilization of enzymes on a hydrophilic surface, to the corresponding method for producing the carriers, and to their use as enzyme immobilizers.

It is known that enzyme immobilization is a now widespread and well-developed technique that allows to use enzymes as biocatalysts even at the industrial level. Immobilization allows, with undisputed advantages for industrial processes, the recovery and recycling of the enzymes after each individual reaction cycle and simplifies enormously the intermediate conditioning processes.

The literature already includes many publications of immobilization protocols.

EP-0065069 discloses latices with a core-shell construction for fixing and immobilizing biologically active substances, which are obtained by adding 0.1 to 20% by weight of a cross-linking agent that can be polymerized radically to functional monomers that are present in a concentration of 4.9 to 99.9% by weight, can be polymerized radically and correspond to the formula Z'-(R)ₙ-X, where Z' is the polymerizable unit, R is the spacer, X is a reactive group, and n = 0.1.

In EP-0200107, proteins, for example enzymes, are adsorbed by an aqueous solution containing an electrolyte in an ionic strength of at least 0.15 mol/l on a macroporous solid carrier, which is wetted by the aqueous solution, is not soluble in water, is formed by small particles and swells weakly at the most; the carrier preferably is not charged or at the most is weakly charged anionically, and preferably before, during or after cross-linked adsorption by means of a coupling component added to the aqueous solution of the electrolyte.

EP-0146329 discloses a process for preparing polymer carriers bearing an oxirane for immobilizing materials bearing active hydrogen, such as for example enzymes. The carriers can be prepared by suspension, by copolymerizing a monovinyl monomer bearing an oxirane group and a trivinyl cross-linking monomer having a hydrophilic character, in the presence of a phase separator that does not react with the oxirane group. The process according to the invention allows to provide carriers in bead form that have high porosity, large surface area, and pores whose diameter is sufficient for the immediate penetration of materials bearing an active hydrogen, such as enzymes.

Immobilizations of enzymes on hydrophobic carriers according to the known art have been devised in the presence of buffered solutions with high ionic strength. The mechanism proceeds by forcing a hydrophobic interaction that moves the biomolecule toward the active groups of the carrier, allowing the formation of covalent bonds, but the limitation consists in the relation between the reactivity of the carrier to nucleophilic agents and the stability in the presence of water, which is in itself a nucleophilic agent; from this standpoint, high stability consequently leads to poor reactivity of the carrier.

One alternative technique that has been developed provides for initial ionic adsorption instead of hydrophobic interaction; in order to ensure hydrophilization of the surface and ionic adsorption it is necessary to introduce additional functional groups, to the detriment of the density of epoxide groups responsible for the formation of the covalent bond.

Moreover, according to such prior art method it is necessary to define a compromise between adsorbent power and availability for the covalent bond; according to the procedures applied so far, the insertion of the adsorbent groups in fact occurs adjacent to the epoxide groups and to their detriment.

Therefore, it is evident that opening too many epoxide groups leads to a limitation of the possibility of establishing a covalent bond; on the other hand, the insertion of a small number of hydrophilizing functional groups neutralizes the adsorbent power of the surface.

US-5,250,613 discloses a method of making a solid surface coated with a hydrophilic outer layer with covalently bonded biopolymers. According to the teachings of US-5,250,613, the total amount of the non-ionic polymer reaction sites have to be used at the same time to bind the polyethyleneimine and the protein.

US-4,772,635 discloses a process for preparing bead-shaped crosslinked copolymers containing epoxide groups and basic amino groups. According to the method disclosed by US-4,772,635, the amines, used as cross-linkers or as modifier to promote a particular pore structure, are introduced before or during the polymerization reaction; acting in this way, the amount of epoxy groups is reduced by their reaction with amino groups and, as a direct consequence, the two different functional groups (amino and epoxy) are one next to the other: therefore, the final result is not a double layer of different active groups where each is responsible for a specific step in the immobilization mechanism.

US-3,932,557 discloses a reactive hydrophilic epoxy containing polymer. The hydrophilic character of the polymer described in US-3,932,557 is given by a polar co-monomer introduced during polymerization; in that case, too, it is possible to observe the formation of two different active groups one next to the other without the presence of any double layer structure.

US-4,210,723 discloses a procedure for obtaining a polymer with a core-shell structure characterized by the presence of active epoxy groups, there is no disclosure of the introduction of other protic functionalities in the chemical structure of the final product to confer a more hydrophilic character while achieving a double layer functionalization responsible for the enzyme immobilization.

US-5,874,165 discloses a method for the immobilization of bioactive species onto polymeric substrates wherein the hydrophilic environment is created by the use of a polymeric material on whose surface an additional hydrophilic polymer is bonded through cross-linking to a surfactant. In this way, a part of the reactive groups of the external polymer is used for the linkage to the first layer consisting of the support member and the cross-linked surfactant.

The aim of the present invention is to eliminate the drawbacks noted above in known types of carrier for immobilizing enzymes, by providing a carrier device for promoting the multipoint covalent immobilization of enzymes on a hydrophilic surface by initial ion absorption followed by the formation of a covalent bond.

Another object of the invention is to provide a carrier in which the availability of the epoxide groups for reaction is higher, since they are spaced from the polymeric matrix, which has no negative effect on their reactivity, as is known in the literature.

An important object of the invention is to allow the formation of multipoint covalent bonds in which a single molecule is immobilized in multiple points by multiple epoxide units; this kind of interaction leads to a strong stabilization of the enzyme.

Another object of the invention is to provide a system for anchoring to the enzyme that is characterized by a good degree of flexibility, minimizing any distortions that might occur during the formation of the covalent bond between the enzyme and the carrier.

This aim and these and other objects are achieved by the carrier according to the invention, which allows multipoint covalent immobilization of enzymes on the hydrophilic surface and is characterized in that it has an inner layer of protic groups and an outer layer that bears epoxide groups.

Conveniently, in the carrier the protic groups that characterize the hydrophilic inner layer and the epoxide groups that characterize the outer surface are arranged on the same hydrocarbon chain, so that one kind of group is not inserted to the detriment of the other, as occurs if they are inserted adjacently.

The particularity of these products and the reason why they can be considered ideal for using and exploiting all the advantages of a combined mechanism of ion absorption and covalent bond with which the enzymes are attracted onto the hydrophilic surface of the carrier and are then bonded covalently consists in having two different functionalizations, each having a role in this mechanism.

Initial ion absorption occurs by means of an innermost layer of protic groups, which can bear charges in operational conditions; after the enzyme has been physically adsorbed in this manner, the outermost epoxide groups are attacked by the nucleophilic agents that are present on the enzyme, forming a covalent bond.

The presence of epoxide end groups allows the formation of multipoint covalent bonds, in which a single molecule is immobilized in multiple points by several epoxide units; this kind of interaction leads to a strong stabilization of the enzyme.

Another aspect of the invention is a process for synthesizing the carrier described above, which comprises the steps of:
a) introducing a protic group on the epoxide end functionality of a compound chosen from the group that comprises a copolymer and a monovinyl monomer by means of a functionalizing reagent;
b) making the protic group introduced on the compound react with bis-epoxide reagents;
c) when the compound is a monovinyl monomer, polymerizing the monomer in suspension by way of a cross-linking agent.

The synthesis strategy allows to insert the epoxide functionalities after the insertion of the adsorbent groups and on the same side chain.

According to this synthesis scheme, the starting material can be constituted by a copolymer as is or by a precursor thereof in the form of a monovinyl monomer that is polymerized at the end of the process. A protic group is introduced on the epoxide functionality of the starting material, and provides the adsorbent characteristics of the carrier with respect to the enzyme to be immobilized. Finally, the protic group is reacted with bis-epoxide reagents, so as to produce epoxide functionalities on the same chain of carbon atoms provided with the protic groups.

Conveniently, when the starting material is a copolymer with epoxide end functionalities, it is synthesized by way of the technique of suspension polymerization starting from a monovinyl monomer that has an epoxide in the end position and by way of a cross-linking agent chosen among the compounds that are known in the art. Preferably, the agent is a bis-vinyl cross-linking agent; preferably, the cross-linking agent is ethylene glycol dimethacrylate.

Advantageously, the protic group introduced on the epoxide end functionality of the starting compound includes a group selected from the class that contains the amine, thiol and hydroxyl groups.

Moreover, in the present synthesis process the stoichiometric ratio between protic functions and the reagent bearing the epoxide group is between 1 and 2; this ratio allows to bind the epoxide functionalities to the adsorbent groups on the same chain of carbon atoms.

Preferably, the stoichiometric ratio between protic functions and the reagent bearing the epoxide group is 1/1.25.

Accordingly, the technical problem that characterizes the background art, i.e., the application of procedures that entail the insertion of the adsorbent groups adjacent to the epoxide groups, to the detriment of the latter, and in practice limits the adsorbent power and the availability for the covalent bond, is overcome by the present invention.

The present synthesis process in fact improves both of the properties that give the product the ability to immobilize the enzyme, by way of a mechanism that provides for initial ion adsorption followed by the formation of a covalent bond, at the same time generating a spacer arm that separates the active functional group from the polymer matrix, so as to minimize all the negative influences that the proximity of the matrix to the reactive center might induce.

In detail, the synthesis proceeds according to the following steps.

The first step consists in attacking the epoxide group with a reagent that can introduce on the polymer a protic group such as a hydroxyl, thiol or amine end group, by virtue of mono or bifunctional reagents.

The various amine reagents used to provide a complete range of products characterized by modulation of application characteristics include NH₄OH (32% aqueous solution) and end diamines spaced by a chain of carbon atoms of various lengths: H₂N(CH₂)ₙNH₂ with n=2-8.

Other reagents used advantageously include ammonia in aqueous solution and NaHS. It is also possible to insert a protic group with any mono or bifunctional compound bearing a nucleophilic protic group capable of opening the epoxide ring (such as derivatives of dithiol, diamine, dialcohols or other monofunctional nucleophilic agents, such as for example H₂O and H₂SO₄).

The next synthesis step, suitable to generate a side chain that bears an adsorbent hydrophilic function (amine group or the other previously cited ones) and an epoxide end function, which is responsible for the formation of the covalent bond with the nucleophilic groups present on the enzyme, provides for the reaction of the protic derivative (amine, thiol or hydroxyl derivative) with diglycidylethers and diglycidylesters or bis-epoxide reagents.

In particular, preferred reagents are bis-diglycidyl end compounds, butanediol diglycidyl ether, ethylene glycol diglycidyl ether, bis-phenol diglycidyl esters, and bis-glycidyl derivatives of glycerol.

The epoxide functionalization can also be inserted by introducing in the protic derivative a suitable linker (for example chosen among carboxyl end diacids), to which a substrate is bonded which bears an epoxide end group (for example glycidol).

This linker group is chosen among the compounds that are known in the art for this purpose; in particular, derivatives that are particularly useful as linker groups are dicarboxylic end acids, their ester derivatives, amides, dianhydrides, the corresponding chlorides of the acids or any form of functionalization that activates the dicarboxylic end acids for esterification with glycidol, diisocyanates and dichloro derivatives.

The bis-epoxide reagents that can be used also modulate to varying extents the properties of the final carrier.

Another embodiment of the method according to the invention provides for the functionalization of the monomers that constitute the polymer and for subsequent suspension polymerization.

In this case, the monomer that bears the epoxide functionality is subjected to all the synthesis steps that have already been described in the case of polymer functionalization.

The same reaction conditions can be applied to a solution of the monomer in an appropriate solvent; isolation of reaction intermediates occurs by distillation and evaporation at reduced pressure of the solvent, of the unreacted initial products and of any reaction byproducts.

Polymerization of the amino-epoxide monomer is performed according to the technique of suspension polymerization with a suitable cross-linking monomer and an adequate porogenic agent, both chosen among those known in the field.

Preferably, the monomer is a bis-vinyl cross-linking monomer; preferably, the cross-linking agent is ethylene glycol dimethacrylate.

Expanding agents that have been used successfully include 1,2-dichloropropane and methyl isobutyl carbinol, but in general any expanding agent known in the art can be used adequately.

Further characteristics and advantages of the invention will become better apparent from the description of a preferred but not exclusive embodiment of the method for synthesizing the carrier for multipoint covalent immobilization of enzymes, illustrated only by way of non-limitative example in the description that follows.

By using as the initial product a copolymer bearing epoxide end functionalities, synthesized with the suspension polymerization technique starting from a vinyl monomer bearing the epoxide functionality and a suitable cross-linking agent, the resulting polymer is suspended in demineralized water (weight ratio of polymer to water = ½) and an aminating agent is added in a ratio of 5 meq/g of dry resin at a temperature of 70 °C. The reaction is maintained in these conditions for 5 hours, at the end of which the aminated polymer is separated from the aqueous phase by filtration.

If diamines are used as reagents, the resulting polymer must be subjected to hydrolysis of the residual epoxide groups. This step is performed by suspending the amine derivative in 10% H₂SO₄ in a ratio of 5 ml/g of dry resin; the resulting suspension is kept under agitation at 50 °C for 5 hours, at the end of which the hydrolyzed polymer is separated from the aqueous phase by filtration and washed with demineralized water until neutral pH.

The amine functions, converted to a sulfate form following the hydrolysis treatment, are regenerated by reacting under agitation an aqueous suspension (polymer/demineralized water = ½) of the polymer hydrolyzed with 5% NaOH until a persistent basic pH of the aqueous phase is achieved.

In this case also, as before the. aqueous phase is separated by filtration and the final amine derivative is washed with demineralized water until neutrality is achieved.

Finally, the conditions of the subsequent reaction phase entail using an aqueous suspension of amine derivative (polymer/demineralized water = ¼), to which the epoxide reagent is added (4.8/(1-W.R./100)meq/g of wet resin) under agitation and at ambient temperature.

The resulting reaction mixture is left to react for 48 hours, after which the final amino-epoxide product is separated by filtration and washed with 4 volumes (bed volume) of demineralized water.

The invention is now described better on the basis of the figures, wherein:
Figure 1 illustrates the insertion of the protic groups in the synthesis of the carrier according to the invention;
Figure 2 illustrates the step of epoxide end functionalization;
Figure 3 illustrates the immobilization of enzymes according to the known art, which provides for hydrophobic interaction;
Figure 4 illustrates the functionalization reactions for obtaining heterofunctional carriers with protic groups inserted adjacent to the epoxide end groups according to the known art;
Figure 5 is a reaction diagram that uses the carriers synthesized according to Figure 4;
Figure 6 illustrates the mechanism for immobilization by using the carriers according to the invention;
Figure 7 shows two charts related to the activity of the supernatant in the immobilization of the β-galactosidase enzyme from A. oryzae according to example 3;
Figure 8 shows two charts related to enzyme activity in the immobilization of the β-galactosidase enzyme from A. oryzae according to example 3;
Figure 9 shows a chart related to the immobilization of the β-galactosidase enzyme from A. oryzae according to example 3;
Figure 10 shows a chart related to the thermal stability of the β-galactosidase enzyme from A. oryzae according to example 3;
Figure 11 shows a chart related to the thermal stability of the β-galactosidase enzyme from A. oryzae as a function of pH according to example 3;
Figure 12 shows a chart related to the thermal inactivation of the derivative of the enzyme and to the thermal stability of the β-galactosidase enzyme from A. oryzae according to example 3.

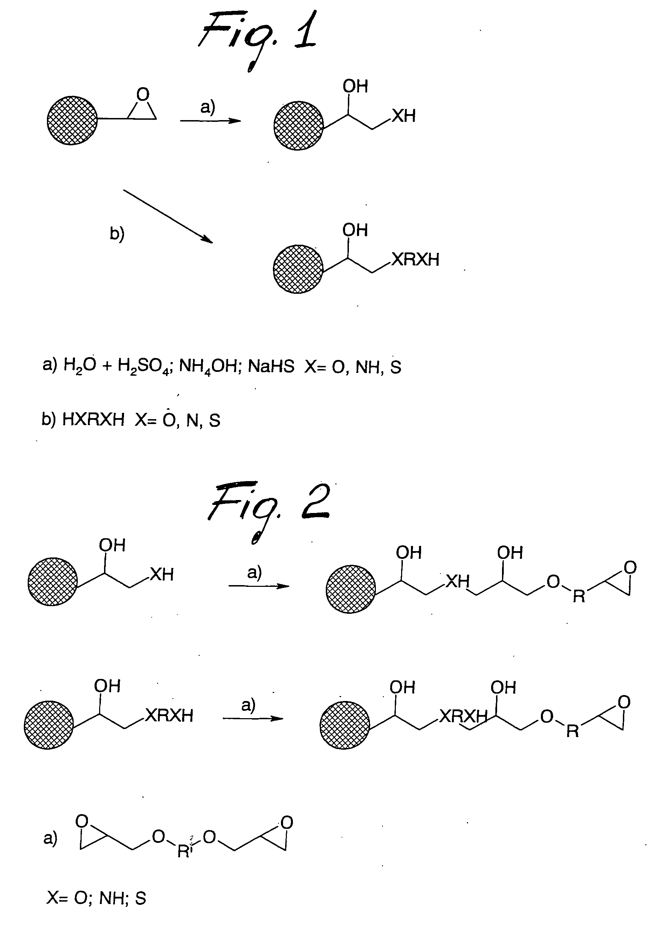

Figure 1 illustrates the step of insertion of the protic groups in the synthesis diagram of the carrier according to the invention. In particular, a protic group is introduced on the epoxide functionality of the starting material and provides the adsorbent characteristics of the carrier with respect to the enzyme to be immobilized. The protic group is introduced with different reagents, as shown by means of the synthesis paths a) and b). In this figure, R can be an entirely generic radical; in the case of diamines, R is (CH₂)ₙ, where n is 2 to 8. Moreover, the correspondence of X with O, NH and S is related to the cited reagents: therefore, if H₂O and H₂SO₄ are used, X is O, et cetera.

Figure 2 illustrates the step of epoxide end functionalization. According to this synthesis diagram, the protic group of the two reaction products shown in Figure 1 (synthesis diagrams a and b) is made to react with the bis-epoxide reagents, so as to produce epoxide functionalities on the same chain of carbon atoms that bears the protic groups. In this case also, R and R' are entirely generic; for example, when using 1,4 butanediol diglycidyl ether, R' is (CH₂)₄.

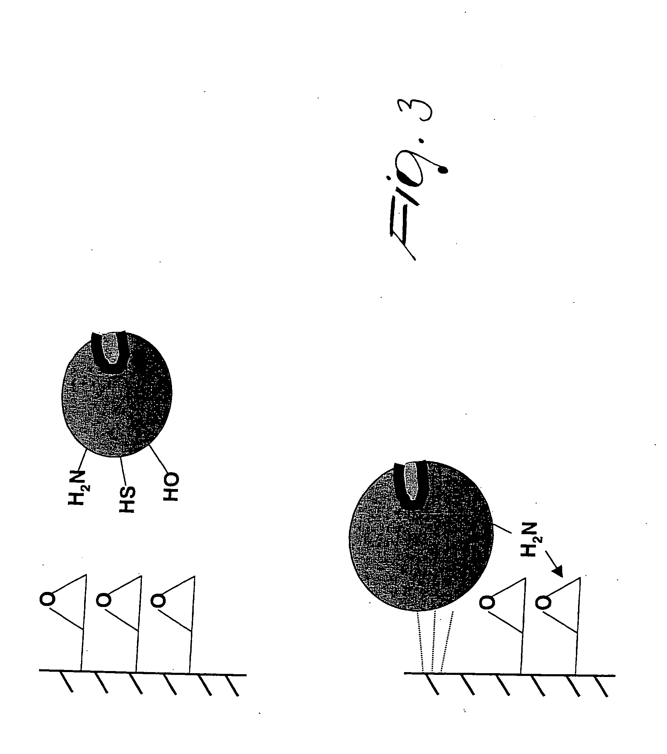

Figure 3 illustrates the immobilization of enzymes on carriers of a conventional type and according to the method that entails forced hydrophobic interaction by using buffers with high ionic strength (e.g. 1.25 M). The covalent intermolecular immobilization process is very slow: at pH 7.0 and at 4-25 °C there is a weak physical adsorption that is hydrophobic in character and is followed by a very fast intramolecular covalent bond.

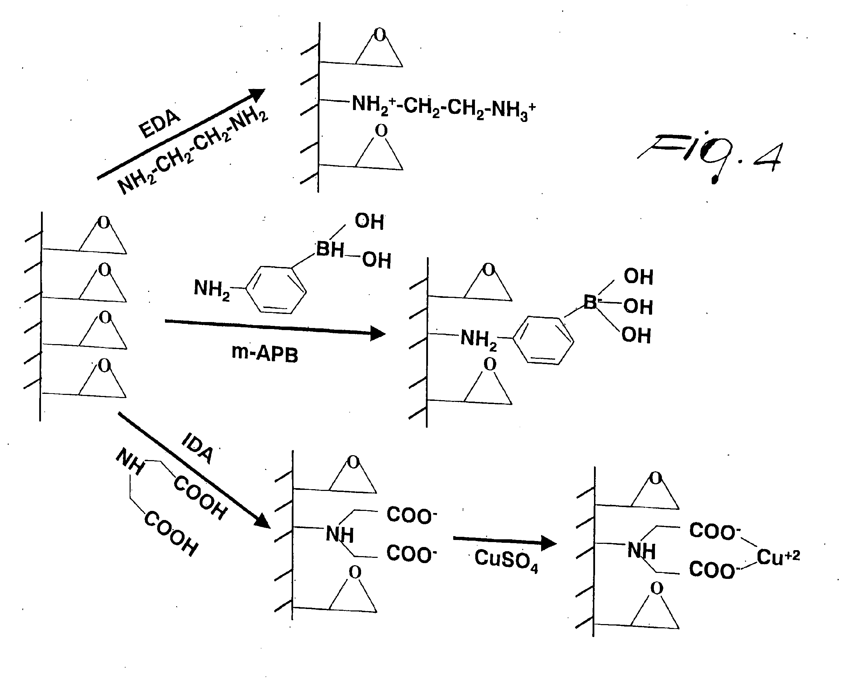

Figure 4 illustrates a functionalization diagram according to the known art, which provides for the insertion of protic groups adjacent to the epoxide functionalities with various amine residues (diamines, aniline derivatives, aminodicarboxylic acids).

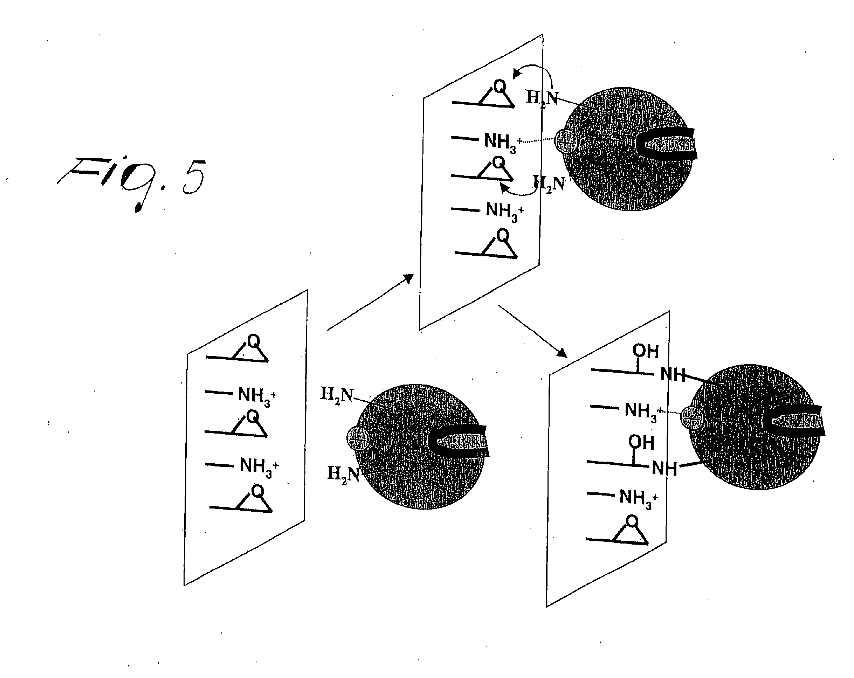

Figure 5 illustrates a diagram of interaction between the carriers synthesized according to the diagram of Figure 4 and the enzyme substrate. The presence, in the matrix of the carrier, of amine groups next to the epoxide groups allows to obtain a physical absorption of the enzyme that is hydrophilic in character. Once adsorbed, the enzyme is immobilized covalently to the carrier by the epoxide groups.

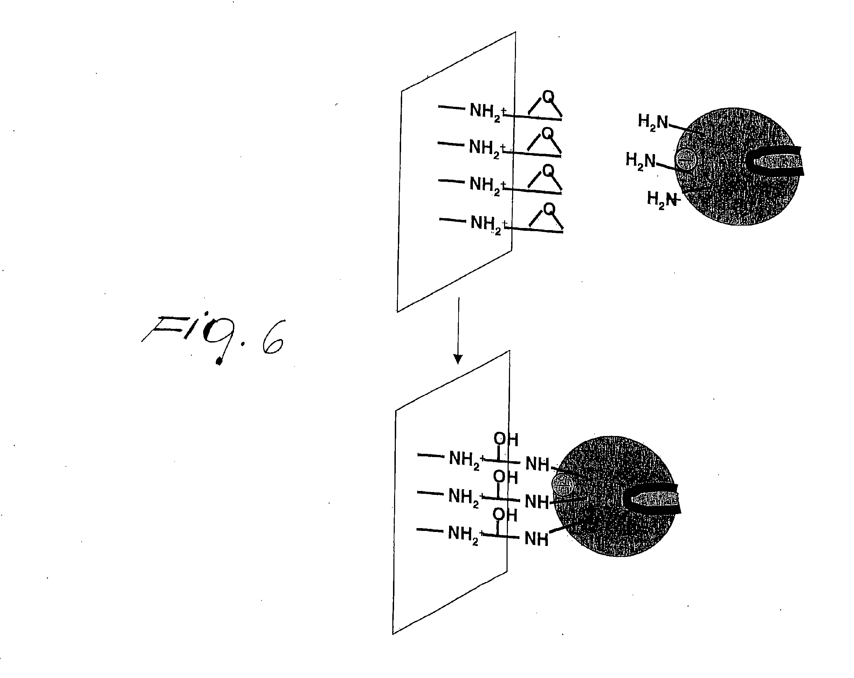

Figure 6 shows how the interaction between the carriers synthesized according to the invention, in which the presence of protic and epoxide groups is evident on the same hydrocarbon chain, allows multipoint covalent immobilization of the enzyme in a two-step mechanism (very fast hydrophilic adsorption and strong covalent immobilization).

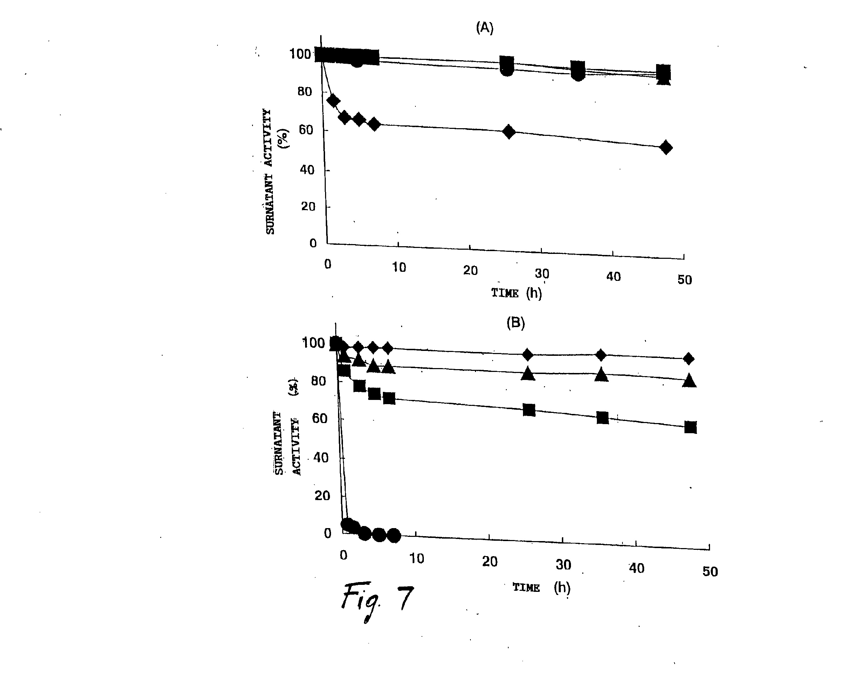

Figure 7 shows the immobilization of β-galactosidase from A. oryzae on Sepabeads EC-EP and Sepabeads EC-EP-LA1 (B) carriers at different ionic strengths: 5 mM (circles), 50 mM (squares), 100 mM (triangles), 1000 mM (diamonds) of sodium phosphate. The enzyme was immobilized at pH 7.0 and 20 °C.

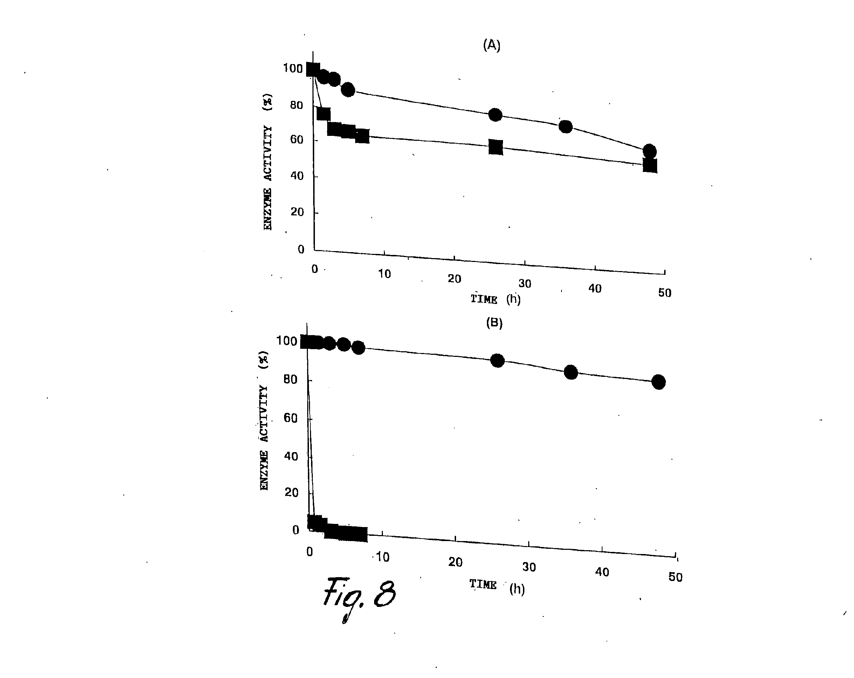

Figure 8 shows the immobilization of β-galactosidase from A. oryzae on Sepabeads EC-EP and Sepabeads EC-EP-LA1 (B) carriers: activity in suspension (circles), supernatant activity (squares). The enzyme was immobilized in sodium phosphate 1M for Sepabeads EC-EP and sodium phosphate 5 mM for Sepabeads EC-EP-LA1. All enzyme immobilizations were performed at pH 7.0 and 20 °C.

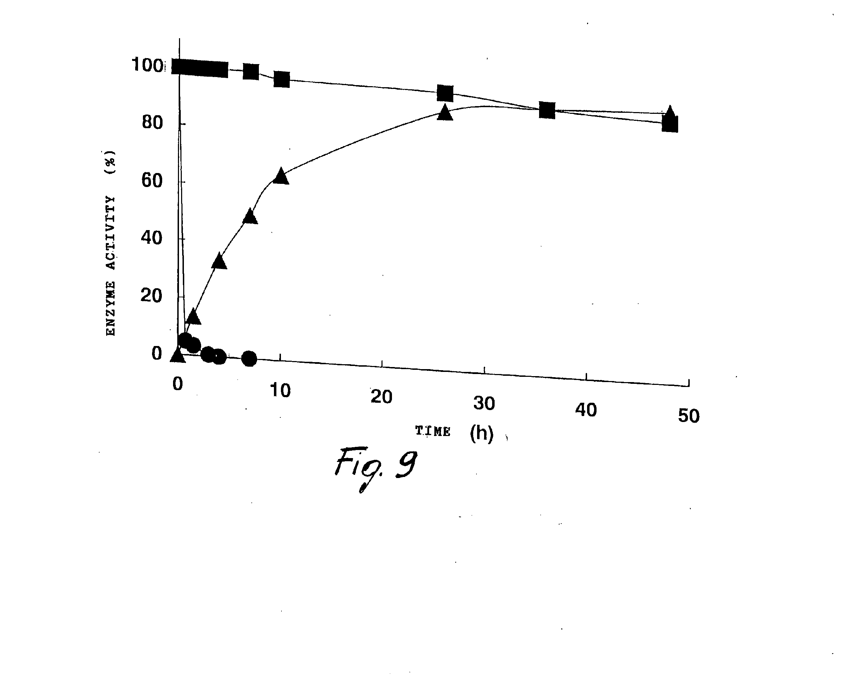

Figure 9 shows the immobilization of β-galactosidase from A. oryzae in sodium phosphate, 5 mM, pH 7.0, 20 °C, as described in the examples. The squares indicate the enzyme in suspension; the circles indicate the enzyme in the supernatant; the triangles identify the covalently immobilized enzyme. This activity of the enzyme was determined by adding 0.5 M of NaCI to a sample of immobilization suspension.

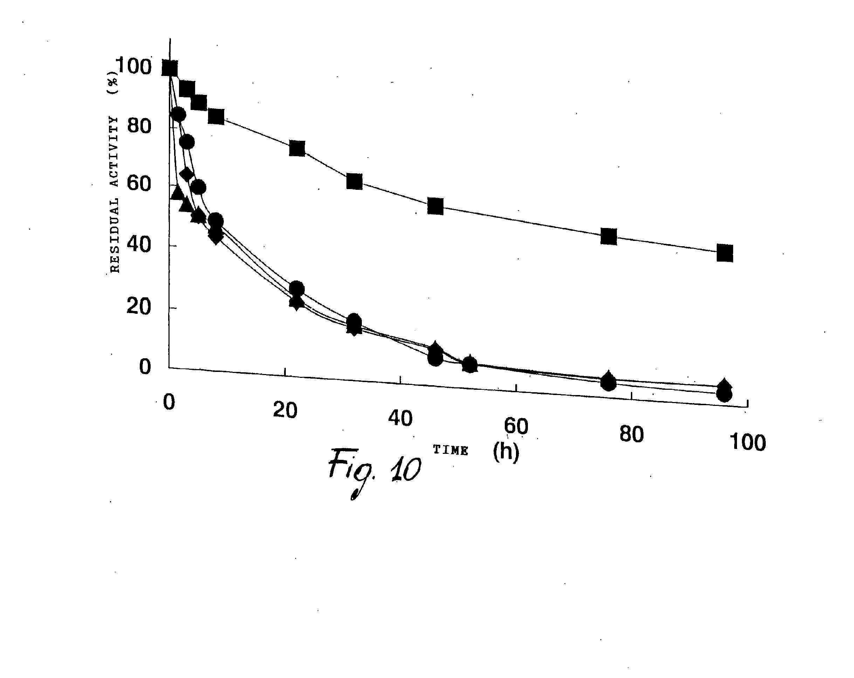

Figure 10 shows the thermal stability of soluble β-galactosidase and of derivatives immobilized on Sepabeads carriers by different methods: soluble enzymes (circles), Sepabeads EC-EP-LA1 (squares), Sepabeads EC-EA (ethylene diamine) (triangles), Sepabeads EC-EA activated with glutaraldehyde (diamonds). Inactivations of derivatives of soluble and immobilized enzymes were performed in sodium acetate, 50 mM, at pH 4.5 and 55 °C.

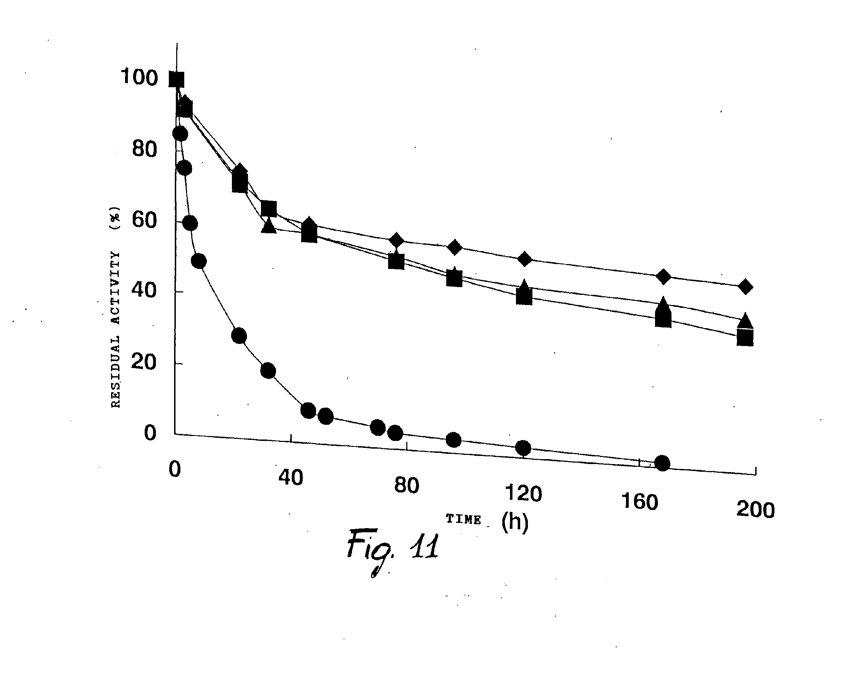

Figure 11 shows the thermal stability of the derivatives of β-galactosidase, immobilized covalently at different pH values on Sepabeads EC-EP-LA1. The enzyme was immobilized beforehand in sodium phosphate, 5 mM, at pH 7.0 and 20 °C over 24 hours, then the pH was brought to alkaline values over 8 hours in order to increase the covalent bond. The circles indicate the soluble enzyme, the squares indicate the enzyme immobilized at pH 7.0, the triangles indicate the enzyme immobilized at pH 7.0 and then incubated at ph 8.5, and the diamonds indicate the enzyme incubated at pH and then incubated at pH 10. Inactivation of the derivatives of the enzyme was performed at pH 4.5 and 55 °C.

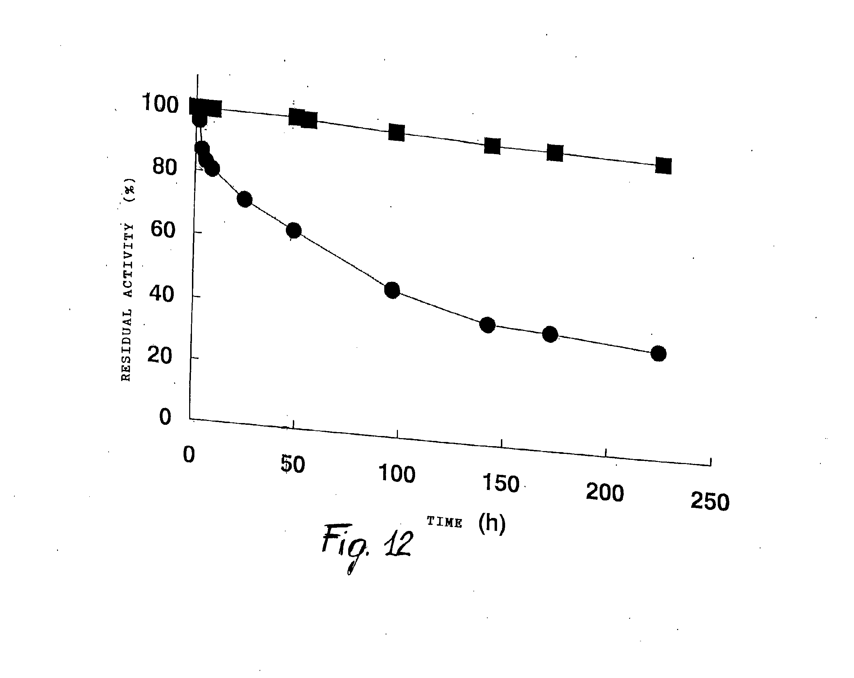

Figure 12 shows the thermal inactivation of the ideal derivative of β-galactosidase in sodium acetate at pH 4.5 and 50 °C. The circles indicate the soluble enzyme, the squares indicate the Sepabeads EC-EP-LA1 derivative. The enzyme was previously immobilized in sodium phosphate, 5 mM, at pH 7.0 and 20 °C over 24 hours and then the pH was brought to 10 over 8 hours in order to increase the covalent bond.

The invention is now also illustrated in a non-limitative manner by way of the following examples.

### EXAMPLE 1

### Synthesis of ethylene diamine derivative (SEPABEADS EC-EA)

1200 g of polymer (water retention = 52.5%) with a methacrylic matrix with epoxide functionality (SEPABEADS EC-EP) are suspended in 2400 ml of demineralized water.

171.3 g of ethylene diamine (5 meq/g dry resin) are slowly added to the resulting suspension, which is kept under agitation by a mechanical agitator (approximately 180 rpm).

The reaction mixture is brought to the temperature of 70 °C and left to react for 5 hours, at the end of which the liquid phase, after cooling to a temperature below 28 °C, is removed by filtration in vacuum with a G0 sintered glass filter.

The polymer is washed with demineralized water until the mother liquors are neutral.

The crude reaction output is hydrolyzed by suspending it in 10% H₂SO₄ and keeping it under agitation (180 rpm) for 5 hours at the temperature of 50 °C.

The hydrolyzed product is then isolated by filtration in the same conditions used previously.

The amine groups are released by treating an aqueous suspension of the polymer isolated in the previous passage with NaOH 5% until the aqueous base is persistently basic.

One obtains 1400 ml of final product by filtration and washing with demineralized water until the mother liquors are neutral.

### EXAMPLE 2

### Synthesis of ethylene diamine, butanediol glycidyl derivative

50 g (Water Retention = 55.5%) of amine derivative (Sepabeads EC-EA) are suspended in 200 ml of demineralized water; 114.8 g of 95% butanediol diglycyl ether (4.8/(1-W.R./100)meq/g wet EC-EA) are added to the resulting suspension, which is kept under agitation at ambient temperature by means of a mechanical agitator (180 rpm).

The reaction proceeds for 48 hours, and the final product is isolated by filtration and washing with 4 volumes (bed volume) of demineralized water.

### EXAMPLE 3

The properties of the carriers for multipoint covalent immobilization according to the invention (Sepabeads EC-EP-LA1) were compared in experiments conducted at the CSIC (Consejo Superior de Investigaciones Cientificas Instituto de Catalisis y Petroleoquimica, Campus Uam, Cantoblanco, Madrid, Spain) with those of conventional carriers (Sepabeads EC-EP), using as a model the β-galactosidase enzyme from *Aspergillus oryzae.*

The new carrier has a layer of epoxide groups over a layer of ethylene diamine that is covalently bonded to the carrier.

### Immobilization of β-galactosidase from A. oryzae on epoxide carriers

5 g of carrier were suspended in 45 ml of solutions of proteins or enzymes (maximum protein concentration: 0.1 mg/ml) in sodium phosphate at pH 7, using different buffer concentrations (from 5 mM to 1 M), at 20 °C. Samples of supernatants were periodically collected and analyzed for protein dosage (Bradford's method; Bradford, 1976) and for enzyme activity. In certain cases, the enzyme was incubated in conditions in which the molecules of the physically adsorbed protein were released, in order to check that the immobilization was covalent. After immobilization, the derivatives were washed in conditions that eliminated any protein molecule that was not covalently attached to the carrier.

### Final point of the enzyme-carrier reaction

To block the epoxide groups completely, 5 g of the carrier or of the enzyme-carrier derivative were incubated in 25 ml of glycin 3M for 16 h at 20 °C. The derivatives were then washed with an excess of distilled water.

### Desorption of proteins that have been adsorbed but not bonded covalently to the carrier

In order to check the covalent bond of the proteins, the conditions of desorption of the proteins adsorbed on the various carriers with the epoxide groups completely destroyed by incubation on 100 mM of sulfuric acid for 24 h were studied.

### Determination of the activity of β-galactosidase from A. oryzae

The activity was analyzed by checking the increase in absorbance at 405 nm caused by hydrolysis of 10 mM of o-nitrophenyl-β-D-galactopyranoside (oNPG) in sodium acetate, 0.1 M, pH 4.6, at 25 °C.

### Analysis of the stability of the enzyme

The derivatives were incubated at pH 4.5 (acetate buffer 50 mM) and at 55 °C; samples were collected periodically and their residual activities were analyzed as described above.

### Immobilizations of β-galactosidase from A. oryzae on Sepabeads EC-EP (A) and Sepabeads EC-EP-LA1 (B) carriers at different ionic strengths (Figure 7): 5 mM (circles), 50 mM (squares), 100 mM (triangles), 1000 mM (diamonds) of sodium phosphate. The enzyme was immobilized at pH 7.0 and 20 °C as described in the examples.

Figure 7 shows the β-galactosidase activity that remains in the supernatant when a solution of the enzyme is incubated in the presence of standard epoxide Sepabeads (Sepabeads EC-EP) and amino epoxide Sepabeads (Sepabeads EC-EP-LA1). Immobilization of the enzyme on conventional carriers requires the use of a high ionic strength. When using the standard epoxide carrier, even at a high ionic strength, the immobilization rate is very low, and it is necessary to use a higher ratio of gel/enzyme solution in order to achieve complete immobilization of the enzyme in 24 h (results not shown).

Anyway, by using the new amino epoxide Sepabeads the dependency of the immobilization rate on the ionic strength is completely reversed; the immobilization rate is higher when the ionic strength decreases. This fact suggests that the immobilization of the enzyme on the carrier follows a mechanism that is completely different from the conventional one. Moreover, the immobilization on the new carriers has shown itself to be extraordinarily fast, very probably because ion adsorption is much faster than hydrophobic adsorption.

### Immobilization of β-galactosidase from A. oryzae on Sepabeads EC-EP (A) and Sepabeads EC-EP-LA1 (B) carriers (Figure 8): activity in suspension (circles), supernatant activity (squares). The enzyme was immobilized in sodium phosphate 1 M for Sepabeads EC-EP and sodium phosphate 5 mM for Sepabeads EC-EP-LA1. All the immobilizations of enzymes were performed at pH 7.0 and at 20 °C as described.

Figure 8 shows the immobilizations of the enzyme on both carriers in their respective ideal conditions. While use of the new carrier left enzyme activity almost unchanged during the immobilization process, most of the enzyme immobilized on the hydrophobic carrier was found to be inactivated. This suggests a different orientation of the molecule of the enzyme, which exposes some catalytically relevant residues to the reaction with the carrier when it is immobilized by means of hydrophobic areas but not when the areas with the highest negative charges are used.

Using epoxide EDA Sepabeads treated with sulfuric acid, the concentration of NaCI required to desorb the enzyme fully from the carrier (500 mM) was determined. Thus, in order to check that the immobilization of the enzyme on the carrier was truly covalent, the derivatives were incubated in 500 mM of NaCI.

### Immobilization of β-galactosidase from A. oryzae on Sepabeads EC-EP-LA1. Figure 9 shows the immobilization of β-galactosidase from A. oryzae in sodium phosphate, 5 mM, at pH 7.0 and at 20 °C as described. The squares indicate the enzyme in suspension; the circles indicate the enzyme in the supernatant; the triangles indicate the covalently immobilized enzyme. This activity of the enzyme was determined by adding 0.5 M of NaCl to a sample of immobilization suspension. This treatment does not release the molecules of the enzyme that are bonded covalently by the carrier. The activity of the supernatant was analyzed subsequently.

Figure 9 shows that while most of the enzyme leaves the supernatant in just a few minutes, a large percentage becomes desorbed from the carrier after incubation with 500 mM of NaCI in the first steps of immobilization, although after 30 h almost all of the enzyme becomes covalently bonded to the carrier. This experiment shows clearly that the enzyme first becomes ionically adsorbed and then the covalent enzyme-carrier bond is established. Bearing in mind that the carrier has a double layer of secondary amine groups, we can associate these amine groups with the behavior of the first rate of immobilization with the ionic strength.

### Thermal stability of soluble β-galactosidase and derivatives thereof immobilized on Sepabeads carriers by various methods (Figure 10): soluble enzymes (circles), Sepabeads EC-EP-LA1 (squares), Sepabeads EC-EA (ethylene diamine) (triangles), Sepabeads EC-EA activated with glutaraldehyde (diamonds). Inactivations of derivatives of soluble and immobilized enzymes were performed in sodium acetate, 50 mM, at pH 4.5 and 55 °C.

Figure 10 shows that the immobilized preparation is much more stable than the soluble enzyme (12 times more) and the freshly adsorbed enzyme (this derivative was in fact found to be only slightly less stable than the soluble enzyme). This preparation of the enzyme is even 15 times more stable than a preparation of the enzyme immobilized on Sepabeads glutaraldehyde (similar to EDA-epoxide, but with more reactive aldehyde groups). Quite interestingly, the inactivation path is clearly multiphasic, since the second enzyme fraction (which is incident for approximately 50% of total activity) is approximately 50 times more stable than the first fraction and approximately 750 times more stable than the soluble enzyme.

Therefore, one should assume that some multipoint covalent bonds can be established between the enzyme and the epoxide amine carrier and are responsible for the increased stability of the immobilized enzyme.

To improve the possibility of increasing the multiple interaction between the enzyme and the carrier, the immobilized preparation was incubated for a further period of 8 h at alkaline pH values.

### Thermal stability of derivatives of β-galactosidase immobilized covalently at different pH values on Sepabeads EC-EP-LA1 (Figure 11). The enzyme was immobilized beforehand in sodium phosphate, 5 mM, at pH 7.0 and 20 °C over 24 hours and then the pH was brought to alkaline values over 8 hours to increase the covalent bond. The circles indicate the soluble enzyme, the squares indicate the enzyme immobilized at pH 7.0, the triangles indicate the enzyme immobilized at pH 7.0 and then incubated at pH 8.5, and the diamonds indicate the enzyme incubated at pH and then incubated at pH 10. inactivation of the enzyme derivatives was performed at pH 4.5 and 55 °C.

Figure 11 shows that when the immobilized enzyme was incubated at alkaline pH values (conditions described previously as convenient for improving the protein-carrier multiple reaction), a fraction of the enzyme (approximately 60% of its activity) becomes much more stable than the derivative that is not incubated at alkaline values of pH.

### Thermal inactivation of the ideal derivative of β-galactosidase in sodium acetate at pH 4.5 and 50 °C (Figure 12). The circles indicate the soluble enzyme, the squares indicate the Sepabeads EC-EP-LA1 derivative. The enzyme had been immobilized beforehand in sodium phosphate, 5 mM, at pH 7.0 and 20 °C over 24 hours and then the pH was brought to 10 over 8 hours to increase the covalent bond.

The epoxide amino Sepabeads carrier is a very interesting alternative to conventional epoxide carriers, first of all because the immobilization mechanism is entirely different (ion adsorption instead of hydrophobic adsorption); secondly, because it should be performed at low ionic strength (the reverse of standard protocols); and finally because the immobilization does not require the use of carriers whose surface has some degrees of hydrophobicity in order to immobilize the enzyme (this can promote some unwanted negative interactions that might cause the instability of the some immobilized enzymes). At pH 7.0, approximately 70-75% of the proteins are immobilized on this new carrier.

In the specific case of β-galactosidase used as a model, it is stressed that the conventional carrier (epoxide Sepabeads) leads to a completely inactive derivative. The heterofunctional amino epoxide carrier has instead allowed to keep quite unchanged the activity of the enzyme during immobilization and has promoted a significant stabilization, which improved in suitable conditions. The ideal derivatives retain their full activity after several days of incubation at 50 °C (see figure), and these conditions are interesting industrially for hydrolyzing lactose in pasteurized milk.

One of the main peculiarities of the present invention, if compared to US-5,250,613, is that all the accessible external reaction sites are functionalized with groups that are able to bind the enzyme, as the epoxy moiety is introduced after an initial functionalization that confers a hydrophilic nature to the surface; in the previously mentioned patent the total amount of the non-ionic polymer reaction sites have to be used at the same time to bind the polyethyleneimine and the protein. Moreover, it has to be pointed out that in the present invention it is truly possible to achieve a real double layer functionalization, while, applying the previously described technique, it is possible to introduce the active group responsible for the protein immobilization next to the functionalities used to bring the hydrophilic nature to the final product.

The main difference between US-4,772,635 and the present invention is that applying the already known procedure the amines, used as cross-linkers or as modifier to promote a particular pore structure, are introduced before or during the polymerisation reaction; acting in this way, the amount of epoxy groups is reduced by their reaction with amino groups and, as a direct consequence, the two different functional groups (amino and epoxy) are one next to the other: therefore, the final result is not a double layer of different active groups where each is responsible for a specific step in the immobilization mechanism.

The hydrophilic character of the polymer described in US-3,932,557 is given by a polar co-monomer introduced during polymerisation; in that case, too, it is possible to observe the formation of two different active groups one next to the other without the presence of any double layer structure.

In US-4,210,723, which describes a procedure for obtaining a polymer with a core-shell structure characterized by the presence of active epoxy groups, there is no disclosure of the introduction of other protic functionalies in the chemical structure of the final product to confer a more hydrophilic character while achieving a double layer functionalization responsible for the enzyme immobilization.

According to US-5,874,165 the hydrophilic environment is created by the use of a polymeric material on whose surface an additional hydrophilic polymer is bonded through cross-linking to a surfactant. In this way, a part of the reactive groups of the external polymer is used for the linkage to the first layer consisting of the support member and the cross-linked surfactant; therefore, only the remaining amount of active groups is free to bind the biomolecule: the differences clearly arise from the comparison with the present technique, where no other polymer except that for the starting material is considered, and where all the external reactive sites are able to bind the biomolecule.

## Claims

1. A carrier for multipoint covalent immobilization of enzymes on a hydrophilic surface, **characterized in that** it has an inner layer of protic groups anc an outer layer provided with epoxide groups.

2. The carrier according to claim 1, wherein the epoxide groups and the protic groups are located on the same carbon atom chain.

3. A method for synthesizing a carrier according to claim 1, comprising the steps of:
a) introducing a protic group on the epoxide end functionality of a compound chosen from the group that comprises a copolymer and a monovinyl monomer by means of a functionalizing reagent;
b) reacting the protic group introduced on said compound with bis-epoxide reagents;
c) when said compound is a monovinyl monomer, polymerizing said monomer in suspension by means of a cross-linking agent and a porogenic agent.

4. The method according to claim 3, wherein in step a) said copolymer is synthesized by way of the method of suspension polymerization, starting from a monovinyl monomer provided with the epoxide functionality with a cross-linking agent.

5. The method according to claim 4, wherein said cross-linking agent is bis-vinyl.

6. The method according to claim 5, wherein said bis-vinyl cross-linking agent is ethylene glycol dimethacrylate.

7. The method according to claim 3, wherein in step a) said protic group is a group selected from the class that contains the amine, thiol and hydroxyl groups.

8. The method according to claim 3, wherein in step a) said functionalizing reagent is mono or bifunctional.

9. The method according to claim 8, wherein said reagent is 32% NH₄OH.

10. The method according to claim 8, wherein said reagent is an end diamine having the formula H₂N(CH₂)ₙNH₂, where n is 2 to 8.

11. The method according to claim 3, wherein in step b) the stoichiometric ratio between the protic groups and the bis-epoxide reagent is between 1 and 2.

12. The method according to claim 11, wherein in step b) the stoichiometric ratio between the protic groups and the bis-epoxide reagent is set at 1/1.25.

13. The method according to claim 3, wherein in step b) said bis-epoxide reagents are selected from the group that consists of diglycidyl ethers and diglycidyl esters.

14. The method according to claim 3, wherein said expanding agent is selected from the group that consists of 1,2-dichloropropane and methyl isobutyl carbinol.

15. The method according to claim 3, wherein step b), after reacting the protic group on the epoxide end compound, comprises the additional steps of:
-- introducing a linker on the protic group;
-- bonding to the linker a substrate provided with an endpoint epoxide group.

16. The method according to claim 15, wherein said linker is selected from the group that consists in carboxyl end diacids, their esters, amides, anhydrides, the corresponding chlorides of the acids, the diisocyanates and the dichloro derivatives.

17. The method according to claim 15, wherein said epoxide end group is glycidol.

18. Use of a carrier according to claim 1 for the multipoint covalent immobilization of enzymes on a hydrophilic surface by way of the ion adsorption of the enzyme and the covalent binding of the enzyme by virtue of the interaction between the surface epoxide groups of the carrier and the nucleophilic agents that are present on the enzyme.
